# EUROPEAN PATENT APPLICATION

(11) **EP 2 446 857 A1**
(43) Date of publication of application: **02.05.2012**
(21) Application number: 10774444.3
(22) Date of filing: 17.03.2010
(51) Int. Cl.: A61F 2/06

(54) **ENDOVASCULAR POSITIONING AND DELIVERY DEVICE**

(30) Priority: 12.05.2009 BR PI0901903
(71) Applicant: Biokyra Pesquisa E Desenvolvimento Ltda, 88036-001 Florianópolis - SC (BR); Lourenço, Marco Antonio, 80250-200 Curitiba - PR (BR)
(72) Inventor: LOURENÇO, Marco Antonio, 80250-200 Curitiba - PR (BR); FACCHINI DE SOUZA, Charles Cristian, 88036-001 Florianópolis SC (BR); PEREIRA, Fernando Marcelo, 88036-001 Florianópolis SC (BR); MASIERO FILHO, Isaias, 88036-001 Florianópolis - SC (BR)
(74) Representative: Isern-Jara, Jaime
(86) International application number: PCT/BR2010/000091
(87) International publication number: WO 2010/130017

(57) **Abstract**

DEVICE FOR ENDOVASCULAR PLACEMENT AND DELIVERY, minimally invasive, percutaneous, endovascular placement device used in placement and/or delivering of other endovascular devices, such as staples, stents or drills, in a blood vessel portion or within a hollow body organ, comprised of delivery device(1) of catheter type, and cylindrical shaped, having a guide-tube(11), one, two, three or "*n*" delivery tubes(12) distributed in a uniform way and circularly around the guide-tube(11), one ogive(14) set at upper end of said guide-tube(11) and articulated stems(13) joining the guide-tube(11) to delivery tubes(12), being the upper end of said stem(13) set into the ogive(14) and the lower end(131) of said stem(13) articulated into the delivery tubes(12) or in support stems.

## Description

It is a minimally invasive, percutaneous, endovascular placement device used in placement and/or delivering of other endovascular devices, such as staples, stents or drills, in a blood vessel portion or within a hollow body organ.

The catheter-type, endovascular placement device, reported herein, may be employed in any type of endovascular application requiring a precise placement associated to a delivery system. A specific application for the endovascutar placement device, reported herein, is using it as a system for placing, delivering and setting up staples or fasteners for vascular grafts on vessel walls, by endovascular suture. This application will be described in detail below. In addition to the application described above, the endovascular placement device, reported herein, nay be used in other surgical applications requiring an adequate placement for delivering specific medical devices.

Hopkinson and Hinchliffe [HINCHLIFFE R. J., HOPKINSON B. R. Current concepts and controversies in endovascular repair of abdominal aortic aneurysms - Journal of Cardiovascular Surgery. # 44, p- 481-502, 2003] describes some types of problems that occur on minimally invasive devices (endo-prosthesis) used in arterial aneurism diseases, highlighting leakages (*endoleaks*) and migration among them.

Migration problems, namely the endo-prosthesis movement or sliding, thus exposing the diseased portion or obstructing the blood flow within the vessel, are common in this type of devices. Such migration problems are reduced by using *barbs* or *hooks* fastened on proximal portion of endo-prosthesis structure. The *barbs* or *hooks* penetrate the vessel walls fastening the device and avoiding its migration. The barbs and hooks generally increase the material volume of endo-prosthesis, which consequently increases the delivery system (catheter) profile of the device, thus excluding its use in certain patients with accentuated tortuosities on arteries. Some migration problems may consequently cause some leakages between artery walls and the graft (*endoleak* Type III).

Other leakage types are described by Hopkinson and Hinchliffe (2003). Such leakages may occur due to sealing problems at the device, it is, the vessel wall does not perfectly fit the endo-prosthesis covering, allowing blood leakage between both surfaces (*endoleak* Type 1). Thus, the diseased portion of the vessel is subjected to blood flow and pressure, which may cause injuries and risks to the patient.

Tout III and Tanner (TOUT III H.H., TANNER H.M. A new vascular endostaple: a technical description - Journal of Vascular Surgery, #34, pp. 565-568, 2001) show alternatives for endo-prosthesis fixation in blood vessels. The authors describe that desirable characteristics of an endovascular fixation system are: a) a flexible, small insertion catheter; the catheter end must be easily maneuverable, b) the artery wall must be easily penetrated, in despite of the calcification level of aortic wall; c) the staple must resist to displacement during the endo-prosthesis whole life cycle and the intraluminal portion of the staple must have a low profile.

The same authors describe a technique for placing endovascular staples in endo-prosthesis. The described technique is to pass a catheter through an insertion sleeve of 13F. Into this catheter an optical fiber is inserted by which orifices are done with laser. The staples are placed on the fiber. Once the graft walls and the vessel walls are perforated, the staple is pushed in order to fill the orifice. The staples comprise stainless steel or nitinol springs, pre-shaped such a way that, at natural state, their geometry is a "spring of spring", it is, a spring constructed from another spring with smaller diameter.

Ohki, Deaton and Condado (OHKI T., DEATON D.H., CONDADO J.A- Aptus Endovascular AAA Repair System. Endovascular Today, pp. 29-36, Nov., 2006) describes the application and results obtained with Parodi's device. According to the authors the device has a delivery system of 16F (external diameter), endovascular staples are made of metallic, biocompatible material with helical format and 4 mm diameter.

Some patent documents also describe devices for endo-prosthesis fixation. Patent document EP1300121, by inventor Parodi, describes the placement of a screw-like staple by means of an endovascular device into a vessel.

Patent request document US2004059344, by same inventor Parodi, describes an improvement on device of previous patent including different types of screws staples and delivery apparatus. Staples, as well as parts of delivery system, may be manufactured of stainless steel or polymeric material. Parodi highlights that a part of the components responsible for addressing the staples on delivery system of the device is manufactured from a shape memory material. The system described in the patent, allows for placing one staple at a time, and it is necessary a repositioning for placing another staple.

On patent request US2007032863, Bolduc describes a device using helical rigid screw-shaped staples that are fastened with torque movement. Staples developed by the inventor are shaped in order to allow endo-prosthesis fixation through a *stent* ring, This fixation with endo-prosthesis may be performed by means of a mechanical, magnetic or chemical coupling.

On patent request US2005004582, Edoga and Richard describe a stapler that uses pre-shaped staples manufactured of nitinol or alloys with shape memory. Staples have various shapes. Delivery system uses a balloon to press the staples exit against vessel walls. Once placed, the balloon is inflated, and staples are pushed in order to perforate the vessel and the graft walls fastening the staple to endo-prosthesis. The stapler works with only one staple at a time, and it may be of single use (only one staple) or it may be loaded with several staples. Even loaded with several staples, repositioning is necessary for placing each staple. On inventions development it is kept a high volume of material on delivery system, resulting in a high profile.

On patent request US20080262597A1, Xiao and Marilla describe an endovascular prosthesis fixation device that also works with helical fasteners or staples. On Xiao's and Marilla's device, staples have not individual controls, it is, all the staples are placed and released simultaneously, making difficult or impossible its effective operation in calcified portions of arteries or in areas with accentuated tortuosities.

On the same patent request US20080262597A1, Xiao and Maxilla describe the staple or fastener as having a *loop* shape with a pointed end, and the other end is rounded, and it may be manufactured from several materials. In another patent request, US20080262596A1, Xiao details other types of staples or fasteners to be used as endo-prosthesis holders. Xiao describes staples that are similar to those described by Edoga and Richard on the patent request US20050004582A1 and also staples that are similar to those described by Parodi on patent request US20D40059344A1.

The endovascular placement device, reported herein, presents an alternative for delivering fixation elements, such as staples and other vascular grafts devices or any type of endovascular application requiring a precise placement associated to a delivery system. The inventive feature is the delivery device construction that allows independent handling for each delivery conduit existing in such device.

The endovascular placement device, reported herein, consists of a body that is an integral part of a catheter. This body, comprising a guide-tube, with an ogive on the end for conducting the catheter along the human body; has at least three internal stems with one of the ends set into the ogive and the other end articulated in a delivery tube. The stems, from the ogive, are axially aligned with the delivery tubes, placed and spaced in a uniform and circular way around the guide-tube. This construction deployment significantly reduces the catheter diameter optimizing its navigability in the blood vessels. The device must have at least one delivery tube, and the other tubes could be replaced with placing stems with geometric shape similar to the delivery tubes. The curved end of delivery tube is made of shape memory material or super resilient material and its ends are radiopaque or may have radiopaque marks in order to facilitate the placement and visualization by fluoroscopy.

The conductor catheter has grips connected to delivery tubes and to the ogive. By manual activation, the grips mount and close the device within the patient and allow the activation of the pushers, existing at internal portions of delivery tubes. The pushers will push the devices that will be delivered by these delivery tubes or allow to place other devices that will be carried or delivered to desired position through the delivery tube.

Opening and closing the device consist in mounting and contracting stems of the ogive and delivery tubes end. When mounted, the ends of the delivery tubes in this device are open, so they rest on the walls of the vessel or graft, applying a light pressure on these walls. When closed, the ogive stems and the ends of delivery tubes are retracted, and stretched, remaining close to guide-tube; so the device is closed, in other words, it is covered by a sleeve that is part of conductor catheter. Each delivery tube is activated independently, according to the relative movement of its respective grip. Delivery tubes may also be activated simultaneously through the grips deployed on conductor catheter.

It is important to remember that this equipment may have one, two, three or "n" delivery tubes fastened in "n" stems of the ogive. At the same time, this device must have at least three stems of the ogive, in order to rest properly into the patient. In case of using only one delivery tube, the rest of delivery tubes will be replaced with support stems connected to respective stems of the ogive.

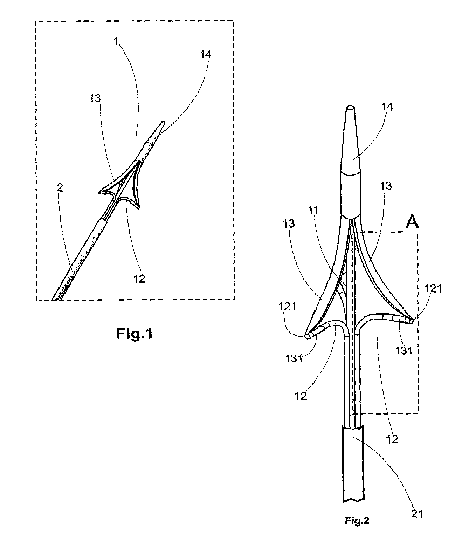

Figure 1 shows global representation of delivery device(1) as an integral part of conductor catheter(2). The conductor catheter(2) is not claimed in present document; the grips belonging to it activate the device(1).
Figure 2 shows a representation of the delivery device(1) when mounted, externally to sleeve(21); the sleeve(21) is an integral part of conductor catheter(2). This delivery device(1) is comprised of a guide-tube(11), delivery tube(12), stems(13) of the ogive(14) and a ogive(14). On configuration represented by figure 2, the delivery device(1), reported herein, has, for functioning explanation purposes, three delivery tubes(12). It is important to highlight that the device(1) could have one, two, three or "n" delivery tubes connected to stems(13) of the ogive(14). In case of being only one delivery tube(12) on device(1) configuration, the rest of delivery tubes(12) will be replaced with support stems that will be used as the necessary support for proper placement of delivery tube(12). On configurations with more than one delivery tube(12), they will be distributed around the guide-tube(11). The guide-tube(11) must allow to freely pass a guide-wire, a known device commonly used in endovascular surgical procedures.

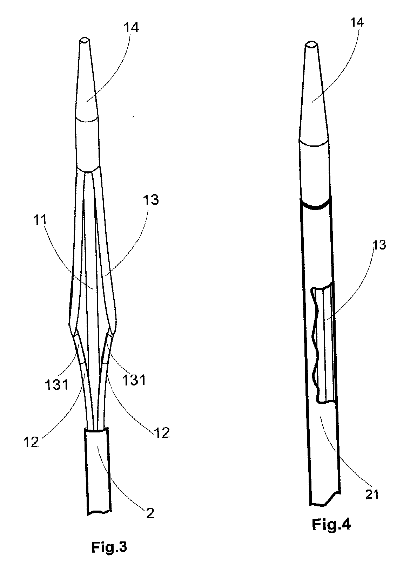

Figure 3 shows a representation of the delivery device(1) on figure 2 when closed and external to sleeve(21);
Figure 4 shows a representation of the delivery device(1), described on figure 2, when closed, inserted into sleeve(21); figure 4 shows the tubular sleeve(21) with a rip to facilitate the visualization of device parts, internally deployed;

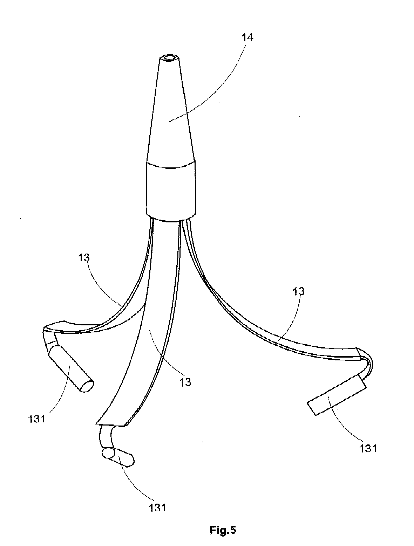

Figure 5 shows a configuration of the device(1) part corresponding to stems(13) of the ogive(14) and the ogive(14). The ogive(14) is fastened to guide-tube(11) end; the upper end of said stems(13) is set into the ogive(14) and the lower end(131) of each stem(13), is articulated and inserted into delivery tube(12) deployed in the same alignment; The set of stems(13) of the ogive(14) and the ogive(14) could be manufactured in a single part, or in separate parts. The stem(13) of the ogive(14) may be manufactured of polymeric or metallic material and the junction with the ogive(14) may be performed by common gluing.

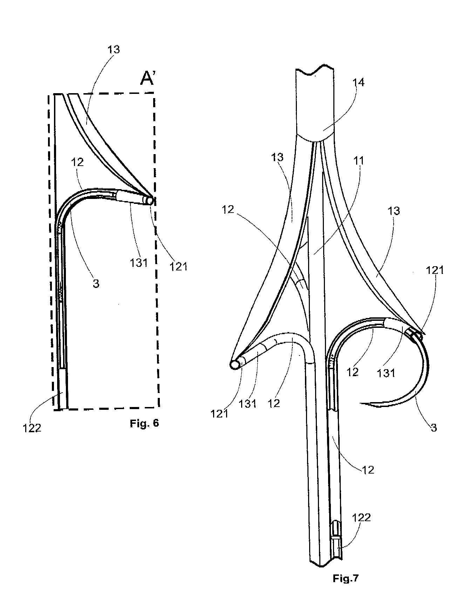

Figure 6 shows the detail 'A' of figure 2, that represents the placement of the staple(3) or other device to be delivered, internally deployed into delivery tube(12) put on a pusher(122) and going out through the end(121) of the delivery tube(12). The staple(3) represented on figure 6 may be the same one described in the patent request by Xiao and Marilla (US20080262597A1), in the patent request by Xiao (US20080262596A1), in the patent request by Edoga and Richard (US20050004582A1) or in the patent request deposited at INPI, Brazil, under the name of *Grampo de Sutura Endovascular* (Endovascular suture staple). The staple(3) represented on figure 6 is not claimed in present document.
Figure 7 schematically shows the exit of a staple(3) when activated its respective activating grip placed into conductor catheter(2) that activates the pusher(122) of delivery tube(12), pushing the staple(3) out of the delivery tube(12). The use of endovascular placement and delivery device with the staples, represented on figures 6 and 7 is only an example of application for the object claimed in present document.

It is important to remember that this device has at least three stems(13) of the ogive(14). Thus, if the device has only one delivery tube(12) connected to one stem(13) of the ogive(14), said device(1) must have two support stems connected to the other two stems(13) of the ogive(14)- If such device has two delivery tubes(12) connected to two stems(13) of the ogive(14), it must have one support stem connected to the third stem of the ogive. The support stem must have geometric shape similar to said delivery tube(12).

Each delivery tube(12) as described on figure 6, has a pusher(122) with independent movement or it could be moved simultaneously with the other ones by means of activating grips placed into conductor catheter(2).

According to showed figures, the placement and delivery device(1) claimed comprise the delivery device(1) activated by a conductor catheter. The conductor catheter is not reported herein.

The delivery device(1) is of catheter type, cylindrical shaped, comprised of a guide-tube(11); one, two, three or "n" delivery tubes(12) distributed in a uniform way and circularly around the guide-tube(11). The delivery device(1) is also comprised of at least three stems(13) of the ogive(14); said stem(13) is made of flexible materials, its upper end is set into the ogive(14) and its lower end(131) is articulated into the delivery tube(12) or in support stems. The set of stems(13) of the ogive(14) and the ogive(14) could be manufactured in a single part, or in separate parts. The stem(13) of the ogive(14) may be manufactured of polymeric or metallic material and the junction with the ogive(14) may be performed by common gluing. Any configuration having stem(13) of the flexible ogive(14) connected, by any means, in one end to the ogive(14) and in the other end to delivery tube(12), made of biocompatible, polymeric or metallic material is object of this request.

The ogive(14) is connected to conductor catheter through guide-tube(11). The conductor catheter, non represented on figures, has grips that activate the delivery tubes(12); and also has a sleeve(2), which is a cylindrical body with the same diameter as the give(14). It may have an inner shape of multi-lumen tube type, through which parts of the device(1), such as delivery tubes(12) and the stems(13) of the ogive (14) are inserted, and through which the guide-tube passes(11). Delivery tube(12) of said device(1) may have a pusher(122) therein, in its lower end, fig. 6 and 7- This pusher(122) is manually activated by means of activating grips placed into conductor catheter. Pushers(122) activation could be done in an independent manner, using one grip for each pusher(122) of each delivery tube(12), or it could be done in a simultaneous manner activating all the pushers(122) of all the delivery tubes(12) simultaneously through the conductor catheter. The end(121) of the delivery tube(12) have radiopaque portions in order to facilitate its visualization by fluoroscopy.

At the moment of introducing the conductor catheter into the human body, the delivery tubes(12) and the stems(13) of the ogive (14) are already inserted into the sleeve(2), in closed mode, fig. 4. The conductor catheter is inserted to reach the placement point, determined with the help of fluoroscopy. Then start the procedure to mount the delivery device(1) and let it placed, in mounted mode, externally to sleeve(2), fig. 3 and 2. In order to mount the device(1), keep the delivery tubes(12) and the stems(13) of the ogive(14) fixed on their places; for this, retain the ogive(14) with the help of the guide-tube(11) and retract back the sleeve(2); thus, the delivery devica(1) is placed in closed mode, externally to sleeve(2), fig. 3. Then, the device(1) is mounted with the help of grips that hold the delivery tubes(12).

## Claims

1. The "DEVICE FOR ENDOVASCULAR PLACEMENT AND DELIVERY", that is an integral part of a catheter, consists of a minimally invasive, percutaneous, endovascular placement device used in placement and/or delivering of other endovascular devices, such as staples, *stents* or drills, among others, in a blood vessel portion or within a hollow body organ, comprising: flexible stems(13), with the upper end set into the ogive(14) and with the lower end(131) articulated into the delivery tube(12) and in which each of the delivery tubes(12), are connected individually and independently to activating grips placed into the catheter;

2. "DEVICE FOR ENDOVASCULAR PLACEMENT AND DELIVERY", according to claim 1, wherein the stems(13) of the ogive(14) and the ends of delivery tubes(12) are retracted and placed, on stretched mode, close to guide-tube(11);

3. "DEVICE FOR PLACEMENT AND DELIVERY OF ENDOVASGULAR DEVICES", according to claim 1, wherein the stem(13) of the ogive(14) and the ogive(14) are manufactured in a single part, from flexible material;

4. "DEVICE FOR ENDOVASCULAR PLACEMENT AND DELIVERY", according to claim 1, wherein the stem(13) of the ogive(14) and the ogive(14) are manufactured in different parts, set into each other;

5. "DEVICE FOR ENDOVASCULAR PLACEMENT AND DELIVERY", according to claim 4, wherein the stem(13) of the ogive(14) and the ogive(14) are manufactured in different parts, and with different materials

6. "DEVICE FOR ENDOVASCULAR PLACEMENT AND DELIVERY", according to claim 1, wherein each of the delivery tubes(12) has an inner pusher(122) and each of said pushers(122) is connected to grips independent from placement catheter, and each of the pushers(122) moves back and forth inside the delivery tube(12) when the operator activates the respective grips of the catheter;
and the ogive(14) is retracted, together with the stems(13) of the ogive(14), fig. 2. The ends(121) of the delivery tubes(12) displace in a radial manner to reach the walls of human organ. Then the placement system is ready to do the delivery through the delivery tube(12). In case of a suture, it is done releasing the staples(3) or fasteners, not included in this request. Staples(3) or fasteners release is done by means of advancing the activating grips, set into conductor catheter, relative to desired delivery tube(12). Activating the proper grips will advance the respective pusher(122) and push the staple(3) or other device to be delivered, such as a *stent* or a drill, out of the delivery tube(12). Figure 7 shows a representation of staple(3) track. The number of staples(3) stored into each delivery tube(12) depends on suture needs evaluated by the specialist. For removing the device(1), it must be closed. The device(1) is closed with the help of grips that retract the delivery tubes(12) retaining the ogive(14), together with the stems(13) of the ogive(14), fig.3. Then retract the set comprised of delivery tubes(12), stems(13) of the ogive(14) and the ogive(14) in order to insert them again into the sleeve(21). Then the device(1) is removed from the patient.

7. "DEVICE FOR ENDOVASCULAR PLACEMENT AND DELIVERY", according to claim 1, wherein each of delivery tubes(12) has radiopaque ends or has radiopaque marks.
